# EUROPEAN PATENT APPLICATION

(11) **EP 2 405 017 A1**
(43) Date of publication of application: **11.01.2012**
(21) Application number: 10168625.1
(22) Date of filing: 06.07.2010
(51) Int. Cl.: C12Q 1/68

(54) **Method for nucleic acid sequencing**

(71) Applicant: Alacris Theranostics GmbH, 68163 Mannheim (DE)
(72) Inventor: Glökler, Jörn, 10783 Berlin (DE); Lehrach, Hans, 14129 Berlin (DE)
(74) Representative: Vossius & Partner

(57) **Abstract**

The present invention relates in a first aspect to a method for sequencing a nucleic acid comprising the following steps: (i) providing a template nucleic acid under conditions at which said template nucleic acid is single-stranded, (ii) annealing a primer oligonucleotide to said template nucleic acid, (iii) ligating or chemically linking said primer oligonucleotide to the template nucleic acid, (iv) contacting said template nucleic acid with a library of adapter oligonucleotides, wherein each adapter oligonucleotide of the library comprises a defined first sequence of two or more nucleotides at one end, a second defined sequence of 12 or more nucleotides at the other end and a random linker sequence of from 2 to 10 nucleotides between the first and second defined sequences, (v) ligating those of the adapter oligonucleotides to the primer oligonucleotide which have hybridized to the template nucleic acid such that their first defined sequence is in immediate proximity to said primer oligonucleotide, (vi) contacting the second defined sequence of the adapter oligonucleotide with a library of labelled oligonucleotide probes such that hybridization of the oligonucleotide probes to complementary strands of said second defined sequences of the adapter oligonucleotides may occur, wherein the label of each oligonucleotide probe is specific for the first defined sequence of the adapter oligonucleotide, (vii) detecting oligonucleotide probes hybridized to said second defined sequences of the adapter oligonucleotide, (viii) removing the linker sequence and the second defined sequence of the adapter oligonucleotide by cleavage of the ligated adapter oligonucleotides between the first defined sequence and the linker sequence or by enzymatic digestion with an exonuclease, (ix) repeating steps (iv) to (viii) as required.

SEQUENCE LISTING

<110>  Max-Planck-Gesellschaft zur Förderung der Wissenschaften
       e.V.

<120>  Method for nucleic acid sequencing

<130>  S1783 EP BLN

<160>  11

<170>  PatentIn version 3.5

<210>  1
<211>  63
<212>  DNA
<213>  Artificial Sequence

<220>
<223>  primer/probe

<400>  1
tgacaccgta cctgctctat gcctatcatg ggcaagcacg ccagggacgt ccctggcgtg      60

ctt                                                                    63


<210>  2
<211>  63
<212>  DNA
<213>  Artificial Sequence

<220>
<223>  primer/probe

<400>  2
tgacaccgta cctgctcttg caataccagg cttaagcacg ccagggacgt ccctggcgtg      60

ctt                                                                    63


<210>  3
<211>  29
<212>  DNA
<213>  Artificial Sequence

<220>
<223>  primer/probe


<220>
<221>  misc_feature
<222>  (3)..(3)
<223>  abasic site

<400>  3
ccntgatagc ggataaagac acagttcgc                                        29


<210>  4
<211>  29
<212>  DNA
<213>  Artificial Sequence

<220>
<223>  primer/probe

<220>
<221>  misc_feature
<222>  (3)..(3)
<223>  abasic site

<400>  4
gcncatgatc gacaatactt gacagcacg                                          29


<210>  5
<211>  29
<212>  DNA
<213>  Artificial Sequence

<220>
<223>  primer/probe


<220>
<221>  misc_feature
<222>  (4)..(4)
<223>  abasic site

<400>  5
gccnatgatc gacaatactt gacagcacg                                          29


<210>  6
<211>  37
<212>  DNA
<213>  Artificial Sequence

<220>
<223>  primer/probe

<400>  6
agagcaggta cggtgtccct atagtgagtc gtattac                                 37


<210>  7
<211>  27
<212>  DNA
<213>  Artificial Sequence

<220>
<223>  primer/probe


<220>
<221>  misc_feature
<222>  (1)..(3)
<223>  n is a, c, g, t or u

<220>
<221>  misc_feature
<222>  (4)..(4)
<223>  abasic site

<220>
<221>  misc_feature
<222>  (5)..(7)
<223>  n is a, c, g, t or u

<400>  7
nnnnnnncga caatacttga cagcacg                                            27


<210>  8

<211>  37
<212>  DNA
<213>  Artificial Sequence

<220>
<223>  primer/probe

<400>  8
agagcaggta cggtgtccct atagtgagtc gtattac                                37


<210>  9
<211>  20
<212>  DNA
<213>  Artificial Sequence

<220>
<223>  primer/probe

<400>  9
cgtgctgtca agtattgtcg                                                   20


<210>  10
<211>  20
<212>  DNA
<213>  Artificial Sequence

<220>
<223>  primer/probe

<400>  10
taatacgact cactataggg                                                   20


<210>  11
<211>  18
<212>  DNA
<213>  Artificial Sequence

<220>
<223>  primer/probe

<400>  11
atagtccctg gcgtgctt                                                     18

## Description

### Field of the invention

This invention is in the field of biology, chemistry, and molecular biology, more in particular in the field of sequencing of nucleic acids.

### Background of the invention

The sequencing of nucleic acids is indispensable for many applications in molecular biology. In particular, nucleic acid sequencing is routinely used in basic and applied research and in forensic, diagnostic, prognostic and screening applications.

Historically there have been two successful approaches to DNA sequence determination: the dideoxy chain termination method, e.g. Sanger et al, Proc. Natl. Acad. Sci., 74: 5463-5467 (1977); and the chemical degradation method, e.g. Maxam et al, Proc. Natl. Acad. Sci. , 74: 560-564 (1977). However, the desire for higher throughputs and more cost-effective sequencing methods has lead to a number of next generation sequencing methods which are for example reviewed in Metzker, Genome Research 15:1767-1776 (2005) and Next-Genration Genome Sequencing, edited by M. Janitz, Wiley-VCH Verlag, Weinheim/Germany, 2008.

Some of the more recent methods are based on a "sequencing by synthesis" approach. Such methods include for instance the pyrosequencing ("454 Sequencing^{™}", Roche Diagnostics) technology which is based on pyrophosphate release, its conversion to ATP and the production of visible light by firefly luciferase (EP 0 932 700 B1; Ronaghi, Genome Research 11:3-11(2001)).

The SOLID^{™} ("Sequencing by Oligonucleotide Ligation and Detection") method (Life Technologies; WO 06/084132 A2) is based on the attachment of PCR amplified fragments of template nucleic acids via universal adapter sequences to magnetic beads and subsequent detection of the fragment sequences via ligation of labelled probes to primers hybridized to the adapter sequences. For the readout a set of four fluorescently labeled di-base probes probes are used. After read-out, parts of the probes are cleaved and new cycles of ligation, detection and cleavage are performed. Due two the use of di-base probes, two rounds of sequencing have to be performed for each template sequence.

Another ligation-based sequencing method is known as CycLiC (Cyclic Ligation and Cleavage). CycLic uses oligonucleotide libraries in which all but one nucleotide is degenerate. The method involves iterative primer extension cycles, base-by-base chain growth by successive ligation and detection steps using labelled oligonucleotides (Mir et al, Nucleic Acids Research 37(1), e5 (2009)).

The Illumina SoIexa® sequencing method is based on sequencing-by-synthesis chemistry (Bentley, Curr Opin Genet Dev. 16(6):545-552 (2006)). Large numbers of unique "polonies" (polymerase generated colonies) are generated that can be simultaneously sequenced. These parallel reactions occur on the surface of a "flow cell" which provides a large surface area for many thousands of parallel chemical reactions.

### Summary of the invention

The present invention provides an alternative sequencing method for nucleic acids with an improved sensitivity, throughput, efficiency and sequencing rate and a large read length.

The present invention relates in a first aspect to a method for sequencing a nucleic acid comprising the following steps:
(i) providing a template nucleic acid under conditions at which said template nucleic acid is single-stranded,
(ii) annealing a primer oligonucleotide to said template nucleic acid,
(iii) ligating or chemically linking said primer oligonucleotide to the template nucleic acid,
(iv) contacting said template nucleic acid with a library of adapter oligonucleotides, wherein each adapter oligonucleotide of the library comprises a defined first sequence of two or more nucleotides at one end, a second defined sequence of 12 or more nucleotides at the other end and a random linker sequence of from 2 to 10 nucleotides between the first and second defined sequences,
(v) ligating those of the adapter oligonucleotides to the primer oligonucleotide which have hybridized to the template nucleic acid such that their first defined sequence is in immediate proximity to said primer oligonucleotide,
(vi) contacting the second defined sequence of the adapter oligonucleotide with a library of labelled oligonucleotide probes such that hybridization of the oligonucleotide probes to complementary strands of said second defined sequences of the adapter oligonucleotides may occur, wherein the label of each oligonucleotide probe is specific for the first defined sequence of the adapter oligonucleotide,
(vii) detecting oligonucleotide probes hybridized to said second defined sequences of the adapter oligonucleotide,
(viii) removing the linker sequence and the second defined sequence of the adapter oligonucleotide by cleavage of the ligated adapter oligonucleotides between the first defined sequence and the linker sequence or by enzymatic digestion with an exonuclease,
(ix) repeating steps (iv) to (viii) as required.

Furthermore, the present invention relates to an oligonucleotide for the sequencing of nucleic acids, wherein the oligonucleotide comprises a defined first sequence of two or more nucleotides at one end, a second defined sequence of 12 or more nucleotides at the other end and a random linker sequence of from 2 to 10 nucleotides between the first and second defined sequences.

In yet another aspect, the present invention relates to a library of oligonucleotides for the sequencing of nucleic acids, wherein each oligonucleotide of the library comprises a defined first sequence of two or more nucleotides at one end, a second defined sequence of 12 or more nucleotides at the other end and a random linker sequence of from 2 to 10 nucleotides between the first and second defined sequences.

The present invention also relates to a kit for the sequencing of nucleic acids comprising in one or more containers:
(i) a library of oligonucleotides according to the invention,
(ii) a library of labelled oligonucleotide probes complementary to said second defined sequences of the oligonucleotides,
(iii) optionally one or more aqueous buffer solutions, e.g. a stripping buffer solution to remove probes after hybridisation and detection,
(iv) optionally an endonuclease, preferably Endonuclease IV, and
(v) optionally a ligase, preferably T4 DNA ligase.

Finally, the present invention also pertains to the use of the method, the oligonucleotide library, the oligonucleotide library and the kit according to the invention for whole genome sequencing or in multiplex sequencing.

### Description of Figures

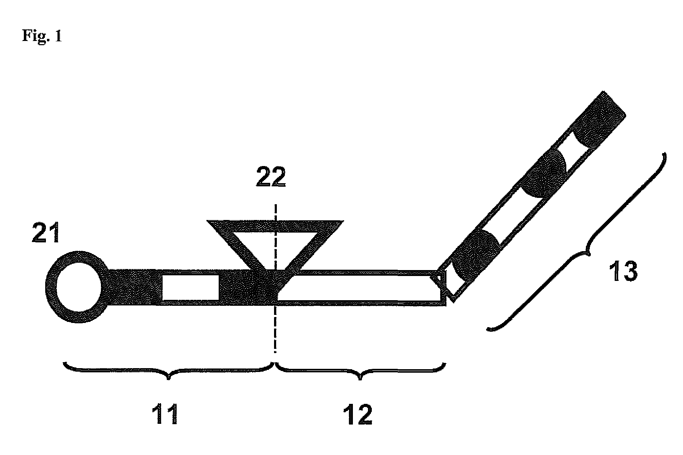

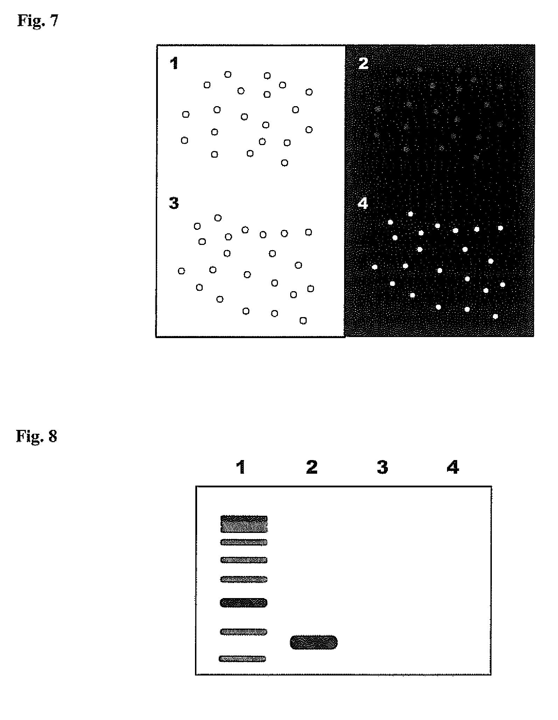

**Fig. 1** schematically illustrates a particular embodiment of the adapter oligonucleotide according to the present invention. The adapter oligonucleotide comprises a first defined sequence (11), a linker sequence (12) and a second defined sequence (13). Further highlighted are the cleavage site (22) between first defined sequence (11) and linker sequence (12) and a phosphate group (21) at the end of the first defined sequence (11).

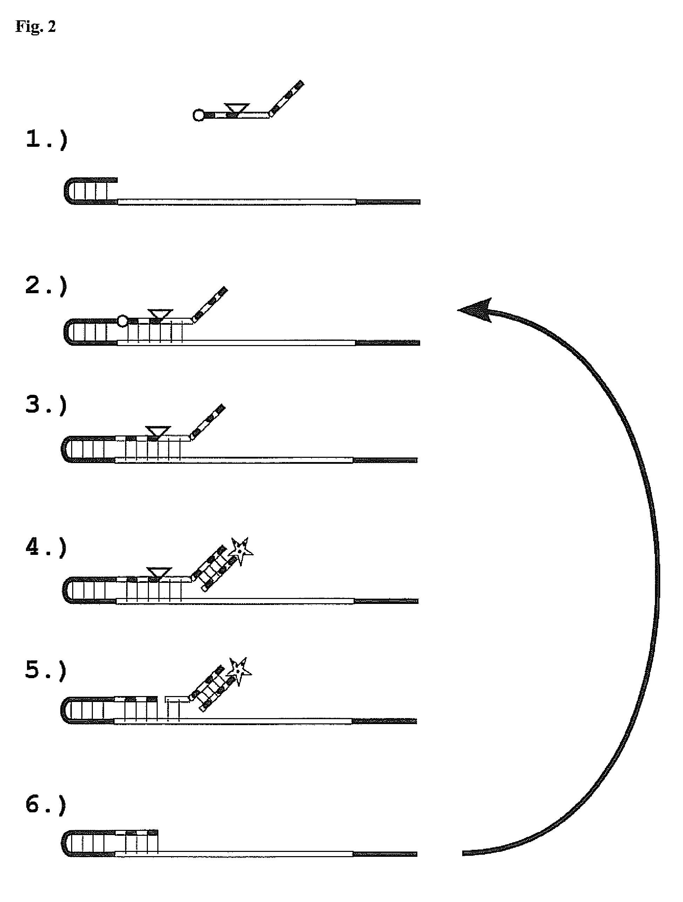

**Fig. 2** illustrates a particular embodiment of the sequencing method according to the invention. 1.) A template nucleic acid having a double stranded region is contacted with adapter oligonucleotides (only one adapter oligonucleotide is shown). 2.) A complementary adapter oligonucleotide hybridizes with its first defined sequence and the linker sequence to the template nucleic acid adjacent to the double-stranded region. 3.) The complementary adapter oligonucleotide is ligated to the end of the double-stranded region of the template nucleic acid. 4.) A labelled oligonucleotide probe hybridizes to the complementary second defined sequence of the adapter oligonucleotide ligated to the template nucleic acid. Subsequently the hybridized oligonucleotide probe is detected. 5.) The adapter oligonucleotide is cleaved between first defined sequence and linker sequence. 6.) Second defined sequence and linker sequence of the adapter oligonucleotide are removed. The first defined sequence remains on the template nucleic acid, thereby elongating its double-stranded region. Subsequently, another cycle may be performed by contacting the template nucleic acid with adapter oligonucleotides.

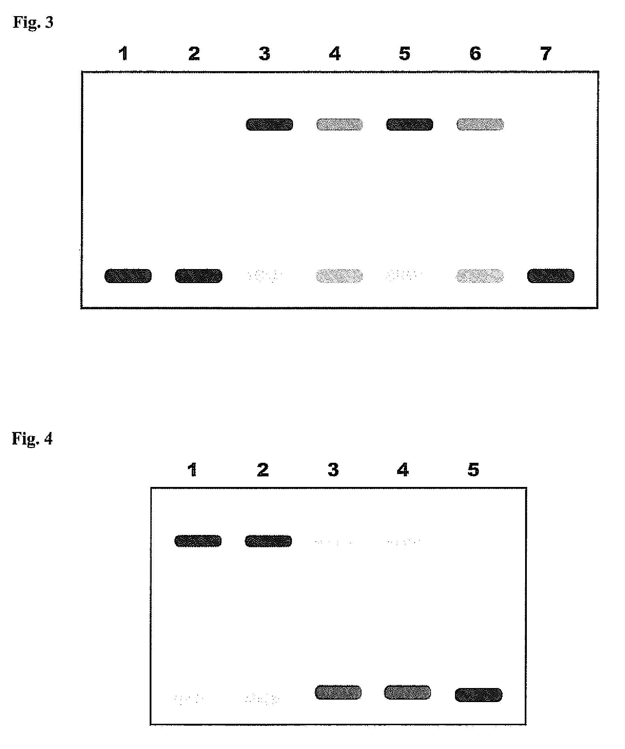

**Fig. 3** denaturing polyacrylamide gelelectrophoresis of different template/oligonucleotide ligations.
   1: control template htmpc with non-matching oligonucleotide a3TOmpi
   2: template htmpc with matching oligonucleotide a3TOmpi without ligase
   3: template htmpc with matching oligonucleotide a4TOmpi ligated at 22°C
   4: template htmpc with matching oligonucleotide a3TOmpi ligated at 20°C
   5: template htmpc with matching oligonucleotide a4TOmpi ligated at 22°C
   6: template htmpc with matching oligonucleotide a3TOmpi ligated at 20°C
   7: template htmpc as a size marker
**Fig. 4** denaturing polyacrylamide gelelectrophoresis of template-oligonucleotide cleavage reactions.
   1: htmpc-a4TOmpi ligation product
   2: htmpc-a3TOmpi ligation product
   3: htmpc-a4TOmpi ligation product after endonuclease IV cleavage
   4: htmpc-a4TOmpi ligation product after endonuclease IV cleavage
   5: template htmpc as a size marker
**Fig. 5** denaturing polyacrylamide gelelectrophoresis of template/oligonucleotide ligation reactions first and second cycle
   1: template htmpc as a size marker
   2: htmpc-a3-rTOmpil ligation product of the second cycle
   3: htmpc-a3-a3TOmpi negative ligation control
   4: htmpc-a3TOmpi ligation product of the first cycle
**Fig. 6** fluorescence microscopy of magnetic beads with DNA template and ligation reaction
   1: htmpi magnetic beads ligated with asNNN and hybridised with C5hGCC under normal light
   2: htmpi magnetic beads ligated with asNNN and hybridised with C5hGCC detected with Cy5 filter by flourescence
   3: htmpi magnetic beads unligated and hybridised with C5hGCC under normal light
   4: htmpi magnetic beads unligated and hybridised with C5hGCC detected with Cy5 filter by fluorescence
   5: htmpi magnetic beads ligated with asNNN without hybridization under normal light
   6: htmpi magnetic beads ligated with asNNN without hybridization detected with Cy5 filter by fluorescence
**Fig. 7** fluorescence microscopy of magnetic beads with DNA template and cleavage reaction
   1: htmpi magnetic beads previously ligated with asNNN, cleaved with endonuclease IV and hybridised with C5hGCC under normal light
   2: htmpi magnetic beads previously ligated with asNNN, cleaved with endonuclease IV and hybridised with C5hGCC detected with Cy5 filter by fluorescence
   3: htmpi magnetic beads ligated with asNNN without endonuclease IV treatment and hybridised with C5hGCC under normal light
   4: htmpi magnetic beads ligated with asNNN without endonuclease IV treatment and hybridised with C5hGCC detected with Cy5 filter by flourescence
**Fig. 8** Iterative ligation and cleavage followed by PCR visualized on agarose gel electrophoresis
   1: 20bp size marker (O'range ruler 20bp, Fermentas)
   2: htmpi magnetic beads after iterative ligation / cleavage with final ligation step
   5: uncoupled magnetic beads without template but with final ligation step
   6: htmpi magnetic beads after iterative ligation / cleavage without final ligation step

### Detailed description of the invention

The present invention provides a new rapid ligation-based nucleic acid sequencing method with high-fidelity. Nucleic acid sequencing according to the present invention is based on hgase-mediated decoding of nucleic acid sequences and oligonucleotide fingerprinting and allows for progressive decoding of long sequences.

According to the method of the invention, primer oligonucleotides are hybridized and subsequently ligated to template nucleic acids to be sequenced. The template nucleic acid is then contacted with a library of oligonucleotide probes ("adapter oligonucleotides") which are allowed to hybridize to the template nucleic acid and subsequently ligated to the primer oligonucleotides if hybridized to the template nucleic acid adjacent to the primer oligonucleotides. Thereafter, labelled oligonucleotide probes are used to detect ligated adapter oligonucleotides. The adapter oligonucleotide have a first sequence for hybridization to the template nucleic acid and a second sequence for detection by the oligonucleotide probes. From the detection of certain bound adapter oligonucleotides one can infer the complementary sequence on the template nucleic acid. After detection, the largest part of the adapter oligonucleotide is removed leaving only the part complementary to the sequence of the template nucleic acid which was actually read-out. Thereafter, further cycles of contacting, ligating, detecting and partially removing the adapter oligonucleotide can be performed to sequence longer consecutive portions of the template nucleic acid.

The present invention, hence, pertains in a first aspect to a method for sequencing a nucleic acid comprising the following steps:
(i) providing a template nucleic acid under conditions at which said template nucleic acid is single-stranded,
(ii) annealing a primer oligonucleotide to said template nucleic acid,
(iii) ligating or chemically linking said primer oligonucleotide to the template nucleic acid,
(iv) contacting said template nucleic acid with a library of adapter oligonucleotides, wherein each adapter oligonucleotide of the library comprises a defined first sequence of two or more nucleotides at one end, a second defined sequence of 12 or more nucleotides at the other end and a random linker sequence of from 2 to 10 nucleotides between the first and second defined sequences,
(v) ligating those of the adapter oligonucleotides to the primer oligonucleotide, which have hybridized to the template nucleic acid such that their first defined sequence is in immediate proximity to said primer oligonucleotide,
(vi) contacting the second defined sequence of the adapter oligonucleotide with a library of labelled oligonucleotide probes such that hybridization of the oligonucleotide probes to complementary strands of said second defined sequences of the adapter oligonucleotides may occur, wherein the label of each oligonucleotide probe is specific for the first defined sequence of the adapter oligonucleotide,
(vii) detecting oligonucleotide probes hybridized to said second defined sequences of the adapter oligonucleotide,
(viii) removing the linker sequence and the second defined sequence of the adapter oligonucleotide by cleavage of the ligated adapter oligonucleotides between the first defined sequence and the linker sequence or by enzymatic digestion with an exonuclease,
(ix) repeating steps (iv) to (viii) as required.

The template nucleic acid is the nucleic acid to be sequenced. It can for example be a DNA, RNA, LNA, PNA or a hybrid of any of these. Typically, however, DNAs or RNAs will be sequenced. Preferably the template nucleic acid is a DNA. The template nucleic acid must be single-stranded at least during certain steps of the method of the invention in order to allow the primer oligonucleotide and the adapter oligonucleotides to hybridize. The template nucleic acid can also be provided in double-stranded form, e.g. it can be double-stranded DNA, if it is subjected to conditions leading to single strands such as elevated ("melting") temperature where appropriate. The template nucleic acids can be of variable lengths. Preferably, they are at least 20 base pairs or nucleotide residues, as the case may be, in length. More preferably the length of the template nucleic acid is between 20 and 1000 nucleotides or base pairs, most preferably between 30 and 200. In the context of the present invention the template nucleic acid can in principle be a single molecule. However, typically the template nucleic acid is a nucleic acid or a plurality of nucleic acids isolated from a biological or forensic sample. The template nucleic acid may have been amplified using suitable amplification methods such as PCR known to the skilled person prior to sequencing. The template nucleic acid may also be a reverse transcript of an RNA. For sequencing, the template nucleic acid may for instance also be provided in fragments of at least 50 base pairs or nucleotide residues in length.

An "oligonucleotide" in the context of the present invention refers to a short nucleic acid polymer, typically with 40 or fewer bases. Although they can in principle be formed by bond cleavage of longer segments, they are typically synthesized by polymerizing individual nucleotide precursors. The nucleotide residues of the oligonucleotides in the context of the present invention may for example be ribonucleotides or desoxyribonucleotides or artificial or modified nucleotides. Some of the oligonucleotides, particularly the adapter oligonucleotides, of the invention may preferably be are chimeric oligonucleotides, i.e. they may comprise different types of nucleotide residues, e.g. DNA and RNA or DNA, RNA and modified RNA (e.g. LNA) nucleotides.

In step (ii) of the method according to the invention, a primer oligonucleotide is allowed to anneal to the template nucleic acid. Annealing refers to the pairing of the primer oligonucleotide by hydrogen bonds to a complementary sequence on the template nucleic acid, forming a double-stranded polynucleotide. Annealing may be facilitated by decreasing the temperature. The primer oligonucleotide typically has a length of from 10 to 40, preferably 15 to 30 nucleotide residues. The sequence of the primer oligonucleotide may be a random sequence. However, in cases were part of the sequence of the template nucleic acid is already known the primer oligonucleotide can be designed to be complementary to such a sequence. In one embodiment, the primer oligonucleotide anneals to either the 3' or the 5' end of the template nucleic acid.
In step (iii) of the method according to the invention the annealed primer oligonucleotide is either ligated or chemically linked to the template nucleic acid. In any case a covalent bond between the primer oligonucleotide and the template nucleic acid is formed. When the primer oligonucleotide is annealed to the 3' or the 5' end of the template nucleic acid and the primer oligonucleotide is ligated to the template nucleic acid by a ligase, a hairpin loop can be formed. T4 DNA ligase may for example be used for this ligation.
Covalent linkage of the primer oligonucleotide to the template nucleic acid can be performed by chemical crosslinking, e.g. using a psoralen. Psoralens are furocoumarins, are a class of organic chemical compounds produced by a variety of plants. Psoralens get activated in the presence of UV-A radiation. They form covalent adducts with pyrimidines. Covalent adducts are formed by linking 3, 4 (pyrone) or 4', 5' (furan) edge of psoralen to 5, 6 double bond of thymine. Psoralens can form two types of monoadducts and one diadduct (an interstrand crosslink) reacting with thymine. The crosslinking reaction by psoralens targets TA sequences intercalating in DNA and linking one base of the DNA with the one below it.

In the context of the present invention, the ligating in steps (iii) and/or (v) is preferably performed using a ligase. A preferred ligase herein is T4 DNA ligase. Other ligases that can be used in the context of the present invention are ligases from the class of ATP-dependent DNA ligases. Ligases that use NAD as cofactor are less preferred. In general, ligases are preferred that are able to recognize and ligate relatively short duplex sequences. T4 DNA ligase is an enzyme that catalyzes the formation of a phosphodiester bond between juxtaposed 5' phosphate and 3' hydroxyl termini in duplex DNA or RNA. This enzyme will join blunt end and cohesive end tennini as well as repair single stranded nicks in duplex DNA, RNA or DNA/RNA hybrids. T4 DNA ligase requires the presence of ATP and Mg²⁺ in the reaction medium. T4 DNA ligase is commercially available.

As outlined above, the template nucleic acid is contacted with a library of adapter oligonucleotides after ligation or cross-linking of the primer oligonucleotide to the template nucleic acid. Each adapter oligonucleotide of the library comprises a defined first sequence of two or more nucleotides at one end, a second defined sequence of 12 or more nucleotides at the other end and a random linker sequence of from 2 to 10 nucleotides between the first and second defined sequences. The first defined sequence serves for the read-out of the complementary positions on the template nucleic acid. The number of nucleotide residues in the first defined sequence hence corresponds to the number of residues which are read-out, i.e. "sequenced", per cycle of the method. The linker sequence also serves for hybridization to the template nucleic acid. Hence first defined sequence and linker sequence of the adapter oligonucleotide serve for the recognition of sequences on the template nucleic acid. First defined sequence and linker sequence together have at least a length of 5 nucleotide residues, preferably between 5 and 20, most preferably between 6 and 15 nucleotide residues. To cover all possible combinations of sequences on the template nucleic acid the library or adapter oligonucleotides must comprise for each possible sequence on the template nucleic acid at least one adapter oligonucleotide which is able to hybridize to the template nucleic acid. The first defined sequence must sample all possible sequence combinations for the nucleotide residues that are to be read-out in a cycle of the method, i.e. 4ⁿ combinations, wherein n is the number of residues to be sequenced in a cycle. The linker sequence, however, may have a degenerate sequence, i.e. the linker sequence preferably consists of degenerate nucleotides. It may for example comprise ambiguous or universal positions that are able to pair with more than one base positions in the template nucleic acid. For example, the linker sequence may comprise inosine bases which are able to base pair with adenine (A), cytosine (C) and uracil (U) or 3-nitropyrrole or 5-nitroindole which are both universal bases. Alternatively, the library can be designed such that it comprises for each and every sequence on the template nucleic acid an adapter oligonucleotide with complementary first defined sequences and linker sequences.
The second defined sequence of the adapter polynucleotides serves for the detection of ligated adapter oligonucleotides. Every single second defined sequence must be uniquely assignable to a distinct first defined sequence and ultimately to the sequence on the template nucleic acid which is to be read-out in a given cycle of the method. In other words, whenever specific second defined sequence is detected this is associated with a template nucleic acid by ligation of the adapter oligonucleotide, this is indicative for the presence of a certain sequence on the template nucleic acid sequence.

The adapter oligonucleotide is designed such that it allows for the removal of its linker sequence and the sequence defined sequence after detection. Thereby, only the first defined sequence of the adapter oligonucleotide remains. The first defined sequence is the sequence which is complementary to the sequence of the template nucleic acid that was read-out in a specific cycle of the method. Removing the linker sequence and the second defined sequence can in principle be achieved in different ways:
a) using an endonuclease that selectively cleaves the adapter oligonucleotide between the first defined sequence and the linker sequence,
b) using an exonuclease that stops cleaving before the first defined sequence, or
c) by selective chemical removal of the linker sequence and the second defined sequence.

Chemical removal can for example be achieved by pH dependent cleavage of RNA. This requires the presence of RNA residues in the linker sequence and the template nucleic acid as well as the primer oligonucleotide and the first defined sequence must not be RNA in this case. RNA can for instance be cleaved at alkaline pH (e.g. around pH 10) while DNA is still stable under these conditions. Hence, in one particular embodiment of the invention cleaving of the adapter oligonucleotide in step (viii) of the method is performed by raising the pH.

In the case an exonuclease, e.g. the Klenow fragment of DNA polymerase I from *E.coli,* is used, the cleavage of the first defined sequence has to be prevented. This can e.g. be achieved by the use of one or more locked nucleic acid (LNA) residues in the first defined sequence, particularly at the penultimate position. The ribose moiety of an LNA nucleotide is modified with an extra bridge connecting the 2' oxygen and 4' carbon. The bridge "locks" the ribose in the 3'-endo (North) conformation. Hence, in a particular embodiment the first defined sequence comprises an LNA nucleotide at the next but one position, i.e. the penultimate position, to the linker sequence.

Different endonucleases may be used for the removal of the linker sequence and the second defined sequence. One possibility is the use of an RNA endonuclease such as RNase H. This would require that the linker sequence comprise ribonucleotide residues adjacent to the first defined sequence. Hence, in one particular embodiment of the method the linker sequence is RNA.

Cleaving of the adapter oligonucleotide in step (viii) is in one preferred embodiment of the invention performed by a endonuclease. The most preferred endonuclease to be used in the context of the present invention is Endonuclease IV. Endonuclease IV is an endonuclease that can act on a variety of oxidative damage in DNA. Endonuclease IV enzyme is an apurinic/apyrimidinic endonuclease that will hydrolyse intact abasic sites in DNA. Abasic sites are cleaved at the first phosphodiester bond that is 5' to the abasic site leaving a hydroxyl group at the 3' terminus and a (deoxy)ribose 5'-phosphate at the 5' terminus. Endonuclease IV from different bacterial sources such as *Th. thermophilus* and *E. coli* can be used. Endonuclease IV enzymes are commercially available. Hence, it is preferred herein that the linker sequence of the adapter oligonucleotide comprises at the position proximate to the first defined sequence an abasic site.

In principle, the sequencing method provided by the present invention works in both reading directions. However, in a particularly preferred embodiment of the invention the sequencing of the template nucleic acid is performed in 3' to 5' direction, i.e. the first adapter oligonucleotide is ligated to the 3' end of the oligonucleotide primer and subsequent adapter oligonucleotides to the 3' end of the respective previous adapter oligonucleotide. This is preferred because most DNA ligases, particularly the preferred T4 DNA ligase, link a free OH groups at the 3' end of a first nucleic acid or oligonucleotide to a 5' phosphate group of a second nucleic acid or oligonucleotide.

However, when the sequencing method according to the invention is performed in the 5' to 3' direction, an additional step of phosphorylating the 5' end of the adapter oligonucleotide after enzymatic cleavage, particularly with the preferred endonuclease IV, is required in order to allow subsequent ligation in the next cycle. Phosphorylation in these cases can for example be achieved by contacting the 5' end of the adapter oligonucleotide with a kinase and ATP. The 5' to 3' sequencing has a better mismatch detection than the 3' to 5' sequencing. The 3' to 5' reading direction is nevertheless preferred because it is less complex than the 5' to 3' sequencing.

In a very particular embodiment of the invention, the primer oligonucleotide is annealed to the 3' end of the template nucleic acid, the first defined sequence is a the 5' end of the adapter oligonucleotide and the sequencing of the template nucleic acid is performed in 3' to 5' direction.

To summarize: 3' to 5' sequencing means that the template nucleic acid is sequenced from 3' to 5' and the adapter oligonucleotide is extended 5' to 3' complementary to the template nucleic acid, thereby forming a double stranded nucleic acid.

The steps of the method of the present invention may require different temperatures such that cycling of the temperature similar as in PCR is required. Particularly the steps in which primers, oligonucleotides or probes are to be annealed or hybridized to another nucleic acid or oligonucleotide may require lowering the temperature to a suitable value which can be calculated by a skilled person according to the length and GC content of the primer probe or oligonucleotide. Hence, the steps (ii), (iv) and/or (vi) of the method of the present invention may require lowering to temperature to allow annealing of complementary oligonucleotides. The method according to the present invention may comprise one or more washing steps to wash away non-hybridized oligonucleotides. This particularly applies before detecting the adapter oligonucleotide with the labelled oligonucleotide probes.

In the context of the present invention, background hybridization can for example be reduced by using spiegelmeric analogues of nucleic acids for detection, i.e. the second defined sequence and the sequence of the adapter oligomers may in a particular embodiment be spiegelmer sequences. Background hybridization can also be reduced by blocking the second defined sequences of the adapter oligonucleotide with complementary oligonucleotides during ligation.

In some very particular embodiments of the invention, the template nucleic acid may be attached to a solid phase, preferably at the end distal from the end to which the primer oligonucleotide anneals. In other particular embodiments the oligonucleotide probes are attached to a solid phase, e.g. (magnetic) beads.

The method of the present invention may be fully or partially automated, e.g. a pipetting roboter and/or a thermocycler may be used. Existing instruments for nucleic acid processing or sequencing, e.g. the Polonator^{™} (Dover Systems, Salem, NH, USA), may be adapted for the present method.

Each oligonucleotide probe in the oligonucleotide probe library is associated with a sequence on the template nucleic acid via the corresponding adapter oligonucleotide. The oligonucleotide probes must be distinguishable by their properties, e.g. their label or other properties such as size, shape, light scattering profile, chemical properties (pH stability, metal binding, other buffer effects), enzymatic activity (release of markers by action of specific enzymes), photostability, marker molecules, holographic encoding (e.g. VeraCode by Illumina Inc.).

The oligonucleotide probes may for instance be labelled with one or more fluorescent dyes or quantum dots. In particular, the oligonucleotide probes of the present invention may be labelled for example by one or more fluorescent moieties which are covalently attached. In the context of the present invention, fluorescent dyes may for example be FAM (5-or 6-carboxyfluorescein), VIC, NED, fluorescein, fluorescein isothiocyanate (FITC), IRD-700/800, cyanine dyes, such as CY3, CY5, CY3.5, CY5.5, Cy7, xanthen, 6-carboxy-2',4',7',4,7-hexachlorofluorescein (HEX), TET, 6-carboxy-4',5'-dichloro-2',7'-dimethodyfluorescein (JOE), N,N,N',N'-Tetramethyl-6-carboxyrhodamine (TAMRA), 6-carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, coumarines such as Umbelliferone, benzimides, such as Hoechst 33258; phenanthridines, such as Texas Red, Yakima Yellow, Alexa Fluor, PET, ethidium bromide, acridinium dyes, carbazol dyes, phenoxazine dyes, porphyrine dyes, polymethin dyes, and the like.

The labelling of the oligonucleotide probes must be such that each individual probe can be distinguished from the other probes since the labelling ultimately corresponds to the sequence on the template nucleic acid. This means that in one embodiment, the number of different labels used equals the number of potential sequences to be read-out on the template nucleic acid. A sequence of two nucleotides to be read-out per cycle requires at least 16 distinguishable oligonucleotide probes. This can be achieved e.g. by using combinations of two or more labels per oligonucleotide probe. Alternatively, the probes can be divided into different subsets which are consecutively contacted with the second defined sequence of the adapter oligonucleotide linked to the template nucleic acid. Each probe in one subset needs to be distinguishable from the other probes in the subset but not necessarily from the probes in the other subsets. Between each addition of a subset the probes of the previous set need to be removed by suitable washing steps.

The library of adapter oligonucleotides may for example comprise at least 16, 64, 256, 1024, 4096 oligonucleotides. This corresponds to 2, 3, 4, 5 or 6 consecutive nucleotides to be read-out per cycle, respectively.

Detection may be performed in the case of fluorescence labels by suitable optical elements, e.g. comprising a camera, a CCD chip, filters fiber optics or the like.

As mentioned herein above, quantum dots may be used as well for labelling of the oligonucleotide probes. This allows the use of more than 4, preferably more than 6, dyes on a single probe.

Fluorescence labels or quantum dots may also be used in conjunction with microspheres, beads, nanoparticles or nanocrystals, for example with the qdots^{™} or qBead^{™} technologies (Eastman et al. (2006), Nano Lett. 6(5): 1059-64; incorporated herein by reference). The oligonucleotide probes may be covalently attached to a microsphere, bead, nanoparticle or nanocrystal which is labelled with fluorescence dyes or quantum dots. Quantum dots provide a nanobarcode which can be read-out to identify the oligonucleotide probe hybridized to the adapter oligonucleotide.

Another possibility of distinguishing individual oligonucleotide probes is to attach each probe to a particle with unique mass and determine the mass by mass spectrometry after binding to the adapter oligonucleotide.

In a further aspect the present invention also pertains to an oligonucleotide for the sequencing of nucleic acids, wherein the oligonucleotide comprises a defined first sequence of two or more nucleotides at one end, a second defined sequence of 12 or more nucleotides at the other end and a random linker sequence of from 2 to 10 nucleotides between the first and second defined sequences. The oligonucleotide of this aspect is the adapter oligonucleotide as defined herein above for the method of the invention.

Furthermore, the invention relates to a library of oligonucleotides for the sequencing of nucleic acids, wherein each oligonucleotide of the library comprises a defined first sequence of two or more nucleotides at one end, a second defined sequence of 12 or more nucleotides at the other end and a random linker sequence of from 2 to 10 nucleotides between the first and second defined sequences. The oligonucleotide library of this aspect is the library of adapter oligonucleotides as defined herein above for the method of the invention.

The invention further pertains to a kit for the sequencing of nucleic acids comprising in one or more containers:
(i) a library of oligonucleotides as defined above,
(ii) a library of labelled oligonucleotide probes complementary to said second defined sequences of the oligonucleotides,
(iii) optionally one or more aqueous buffer solutions, e.g. a stripping buffer solution to remove probes after hybridisation and detection,
(iv) optionally an endonuclease, preferably Endonuclease IV, and
(v) optionally a ligase, preferably T4 DNA ligase.

Finally, the invention also relates to the use of the method according to the invention, or the oligonucleotide or the oligonucleotide library according to the invention or the kit according to the invention for whole genome sequencing, in multiplex sequencing, or in site-specific sequence detection on planar surfaces (*in situ* sequencing).

### Examples

The oligonucleotides and template nucleic acids used in this example are given in Table 1.

### Enzymes:

- Endonuclease IV, 10 U/µl, BioLabs, New England Biolabs
- T4 DNA ligase, 268 U/µl, Roboklon, Berlin, Germany
- Magnetic beads, Agowa genomic, Berlin, Germany
- Carboxyl magnetic beads (Ø 0.5 µm, 600 µmol COOH/g) obtained from Chemicell, Berlin, Germany

| | | | |
|---|---|---|---|
| Ligation: | program: | 94°C | 3 min |
| | | 37°C | 5 min |
| | | 22°C | 30 min |
| | | 80°C | 10 min |

The ligation reaction in solution contained 250 pmol of template nucleic acid (htmpi), 250 pmol of adapter oligonucleotide with an abasic site (see table below), 335 Units of T4 DNA ligase in 10x T4 buffer in a final volume of 50µl. The mix was incubated at 22°C for 30 min. The T4 DNA ligase was inactivated by incubation at 80°C for 10 min.
In the second cycle the materials from the previous reaction were supplemented with 100 pmol of complementary oligonucleotide rTOmpi1, 268 Units of T4 DNA ligase in 10x T4 buffer.

Alternatively, the ligation reaction was performed with coupled Beads contained 108 Beads coupled with 285 ng of template nucleic acid, 617.5 ng of adapter oligonucleotides and 536 Units of T4 DNA ligase in T4 buffer in a final volume of 50µl.

**Table 1: Nucleic acids and oligonucleotides used in Example 1**

| **Name** | **Sequence (5'→3')** |
|---|---|
| ***Template nucleic acids:*** | |
| htmpi (hairpin template sequence) SEQ ID NO. 1 | (C₆NH₂)-TGACACCGTACCTGCTCT**ATGCCTATCATGGGC***AAGCACGCCAGGGACGTCCCTGGCGTGCTT* |
| htmpc (negative control, hairpin seq. control) SEQ ID NO. 2 | (C₆NH₂)-TGACACCGTACCTGCTCT**TGCAATACCAGGCTT***AAGCACGCCAGGGACGTCCCTGGCGTGCTT* |

| ***Adapter oligonucleotides:*** | |
|---|---|
| rTOmpi1 (2^{nd} cycle) (oligo with RNA/riboC) SEQ ID NO. 3 | PO₄-**CC**ΦTGATAGCGGATAAAGACACAGTTCGC |
| a3Tompi (1^{st} cycle) (abasic third position) SEQ ID NO. 4 | PO₄-**GC**ΦCATGATCGACAATACTTGACAGCACG |
| a4TOmpi (abasic fourth position) SEQ ID NO. 5 | PO₄-**GCC**ΦATGATCGACAATACTTGACAGCACG |
| ligT7Pro5 SEQ ID NO. 6 | PO₄-AGAGCAGGTACGGTGTCCCTATAGTGAGTCGTATTAC |
| asNNN (control of ligation) SEQ ID NO. 7 | PO4 -NNNΦNNNCGACAATACTTGACAGCACG |
| ligT7Pro5 SEQ ID NO. 8 | PO4-AGA GCA GGT ACG GTG TCC CTA TAG TGA GTC GTA TTA C |

| ***Oligonucleotide probes:*** | |
|---|---|
| Cy5hGCC (Cy5 hybridisation oligo as as control for GCC ligation) SEQ ID NO. 9 | Cy5-CGTGCTGTCAAGTATTGTCG |

| *PCR Primers:* | |
|---|---|
| T73G (control primer for control PCR) SEQ ID NO. 10 | TAATACGACTCACTATAGGG |
| Pro3 SEQ ID NO. 11 | ATAGTCCCTGGCGTGCTT |

| | |
|---|---|
| - Template nucleic acids: comprising a double-stranded hairpin (italic); sequence to be sequenced in bold; C₆NH₂: - Adapter oligonucleotides: Φ: abasic site; N: any nucleotide, random; first defined sequence in bold; second defined sequence underlined; PO₄: 5' phosphate group. Oligonucleotide probes: Cy5: fluorescent dye | |

| | | | |
|---|---|---|---|
| Cleavage: | program: | 95°C | 3 min |
| | | 37°C | 5 min (0.2°C/s) |
| | | 22°C | 1 h |
| | | 80°C | 20 min |

The cleavage reaction contained the previous ligation reaction, 40 Units of Endonuclease IV in 10x NEBuffer3. The mix was incubated at 22°C for 1 h. The Endonuclease IV was inactivated by incubation at 80°C for 20 min.

### Bead coupling of template nucleic aid:

10 mg of carboxyl magnetic particles washed 2x with 1 ml MES buffer, pH 5. Separate by using the magnetic separator. After second wash step resuspend the particles in 0.25 ml MES buffer containing 10 mg EDC, mix on shaker for 10 min at room temperature. Add 46.92 µg amine group containing template nucleic acid to the particles. Mix on a shaker for 2 h at room temperature. After the incubation wash the particles 3x with 1 ml storage buffer (1x PBS, 0.1% Tween, 10 mM Ethanolamine) or (1x PBS, 0.1% Tween, 10 mM Ethanolamine)

### Hybridisation:

The hybridisation reaction contained the previous ligation or cleavage reaction, 261.4 ng of specific 5'fluorescein labelled oligonucleotide probe (Cy5hGCC) in 10x hybridisation buffer, pH 7,4 (200 mM Tris, 50 mM MgCl₂, 20 mM CaCl₂, 20 mM KCl, 1400 mM NaCl, 0,5% Tween 20). The mix was incubated at 40°C for 5 min. After hybridisation wash 2x with 10x hybridisation buffer and elute in 20µl 1 x storage buffer.

### Ligation with specific oligos

The ligation of free template nucleic acids with specific adapter oligonucleotides has been visualised by PAGE. The resulting products can be identified by a pronounced shift in size; see Fig. 3.

The subsequent cleavage reaction was traced by PAGE and seen as a decrease in product size; see Fig. 4.

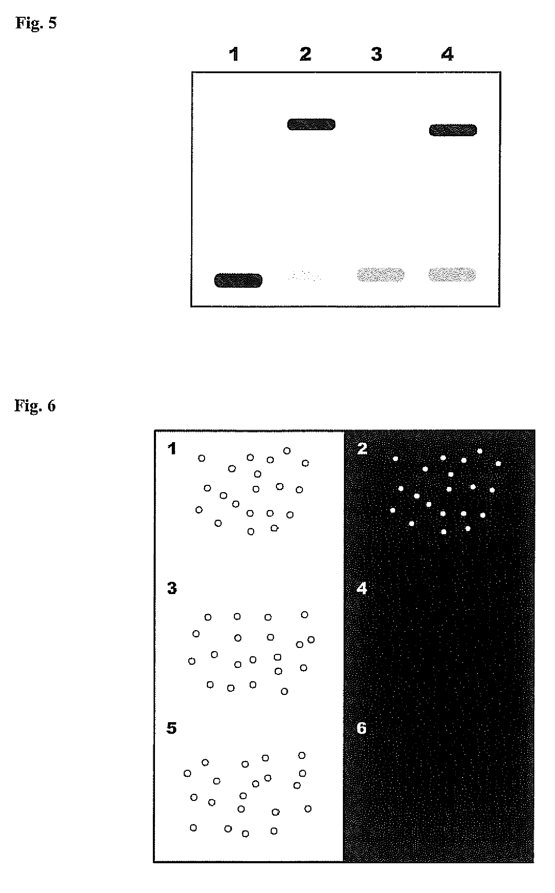

The results from the second cycle were again visualised by PAGE; see Fig. 5.

### Visualisation by hybridization and microscope

The template nucleic acid was coupled covalently to magnetic beads to allow a simple handling of the ligation/cleavage products. Magnetic beads could be visualised by a microscope; see figure 6 for the ligation results. The cleavage reaction was monitored under microscope; see Fig. 7:
Iterative ligation and cleavage followed by PCR. The PCR was conducted for 15 cycles with T73G and Pro3 primers and the resulting product was visualized on agarose gel; see Fig. 8: The amplified fragment was found as expected at 70 bp.

## Claims

1. A method for sequencing a nucleic acid comprising the following steps:
(i) providing a template nucleic acid under conditions at which said template nucleic acid is single-stranded,
(ii) annealing a primer oligonucleotide to said template nucleic acid,
(iii) ligating or chemically linking said primer oligonucleotide to the template nucleic acid,
(iv) contacting said template nucleic acid with a library of adapter oligonucleotides, wherein each adapter oligonucleotide of the library comprises a defined first sequence of two or more nucleotides at one end, a second defined sequence of 12 or more nucleotides at the other end and a random linker sequence of from 2 to 10 nucleotides between the first and second defined sequences,
(v) ligating those of the adapter oligonucleotides to the primer oligonucleotide which have hybridized to the template nucleic acid such that their first defined sequence is in immediate proximity to said primer oligonucleotide,
(vi) contacting the second defined sequence of the adapter oligonucleotide with a library of labelled oligonucleotide probes such that hybridization of the oligonucleotide probes to complementary strands of said second defined sequences of the adapter oligonucleotides may occur, wherein the label of each oligonucleotide probe is specific for the first defined sequence of the adapter oligonucleotide,
(vii) detecting oligonucleotide probes hybridized to said second defined sequences of the adapter oligonucleotide,
(viii) removing the linker sequence and the second defined sequence of the adapter oligonucleotide by cleavage of the ligated adapter oligonucleotides between the first defined sequence and the linker sequence or by enzymatic digestion with an exonuclease,
(ix) repeating steps (iv) to (viii) as required.

2. The method according to claim 2, wherein the linker sequence of the adapter oligonucleotide comprises an abasic site at the position proximate to said first defined sequence.

3. The method according to any of the preceding claims, wherein the primer oligonucleotide is annealed either the 3' or the 5' end of the template nucleic acid.

4. The method according to claim 3, wherein in step (iii) a hairpin loop is formed.

5. The method according to any of the preceding claims, wherein the first defined sequence comprises an LNA nucleotide at the next but one position to the linker sequence.

6. The method according to any of the preceding claims, wherein the template nucleic acid is a DNA.

7. The method according to any of the preceding claims, wherein the primer oligonucleotide has a length of from 10 to 40 nucleotide residues.

8. The method according to any of the preceding claims, wherein the sequencing of the template nucleic acid is performed in 3' to 5' direction.

9. The method according to any of the preceding claims, wherein cleaving of the adapter oligonucleotide in step (viii) is performed by Endonuclease IV.

10. The method according to any of the preceding claims, wherein ligating in steps (iii) and/or (v) is performed using a T4 DNA ligase.

11. The method according to any of the preceding claims, wherein the oligonucleotide probes are labelled with one or more fluorescent dyes or quantum dots.

12. An oligonucleotide for the sequencing of nucleic acids, wherein the oligonucleotide comprises a defined first sequence of two or more nucleotides at one end, a second defined sequence of 12 or more nucleotides at the other end and a random linker sequence of from 2 to 10 nucleotides between the first and second defined sequences.

13. A library of oligonucleotides for the sequencing of nucleic acids, wherein each oligonucleotide of the library comprises a defined first sequence of two or more nucleotides at one end, a second defined sequence of 12 or more nucleotides at the other end and a random linker sequence of from 2 to 10 nucleotides between the first and second defined sequences.

14. A kit for the sequencing of nucleic acids comprising in one or more containers:
(i) a library of oligonucleotides according to claim 14,
(ii) a library of labelled oligonucleotide probes complementary to said second defined sequences of the oligonucleotides,
(iii) optionally one or more aqueous buffer solutions, e.g. a stripping buffer solution to remove probes after hybridisation and detection,
(iv) optionally an endonuclease, preferably Endonuclease IV, and
(v) optionally a ligase, preferably T4 DNA ligase.

15. Use of the method according to any of claims 1 to 11, an oligonucleotide according to claim 12, an oligonucleotide library according to claim 13 or a kit according to claim 14 for whole genome sequencing, in multiplex sequencing, or in site-specific sequence detection on planar surfaces (*in situ* sequencing).
